# EUROPEAN PATENT SPECIFICATION

(11) **EP 4 656 125 B1**
(45) Date of publication and mention of the grant of the patent: **09.09.2026**
(21) Application number: 25163651.0
(22) Date of filing: 13.03.2025
(51) Int. Cl.: A61B 5/00, A61B 5/145, G16H 40/67

(54) **METHOD OF PROVIDING NOTIFICATION OF SIGNAL LOSS IN GLUCOSE MONITORING SYSTEM**
VERFAHREN ZUR BEREITSTELLUNG EINER BENACHRICHTIGUNG ÜBER SIGNALVERLUST IN EINEM GLUCOSEÜBERWACHUNGSSYSTEM
PROCÉDÉ DE FOURNITURE DE NOTIFICATION DE PERTE DE SIGNAL DANS UN SYSTÈME DE SURVEILLANCE DE GLUCOSE

(30) Priority: 28.05.2024 KR 20240069510
(43) Date of publication of application: 03.12.2025
(73) Proprietor: I-SENS, INC., Seoul 06646 (KR)
(72) Inventor: KIM, Kyu Jin, Incheon (KR)
(74) Representative: HGF

(56) References cited:
- AU-A1- 2021 342 491
- CN-A- 116 052 822
- US-A1- 2015 039 054
- US-A1- 2021 322 676

## Description

### BACKGROUND OF THE INVENTION

### 1. Field

Embodiments of the present disclosure relate to providing a notification of signal loss in a glucose monitoring system, and more specifically, to a technology for providing a notification of signal loss when data transmission and reception between a sensor transmitter and a terminal is stopped due to a communication failure or signal loss.

### 2. Description of the Related Art

Document US 2015/039054 discloses a method of providing a notification of signal loss in an implantable medical device. This document discloses that sensor information is transmitted between an implantable medical device (IMD) and an external device, and that first and second communication failure signals exist between the IMD and a remote station.

Recently, with the advancement of medical technology, various medical devices that are attached to a user's body have been developed and sold. A medical device attached to the user's body may be useful for monitoring biometric information or providing treatment by being attached to the skin of a chronic disease patient.

For example, chronic diseases such as diabetes require continuous management, and a medical device that is attached to the skin and measures glucose may be used to monitor glucose in diabetic patients. Diabetes is characterized by almost no noticeable symptoms in the early stages, but as the disease progresses, symptoms specific to diabetes appear, such as polydipsia, polyphagia, polyuria, weight loss, general malaise, itchy skin, and wounds on the hands and feet that do not heal and persist for a long time. As diabetes progresses further, complications such as vision impairment, high blood pressure, kidney disease, stroke, periodontal disease, muscle spasms, neuralgia, and gangrene appear. In order to diagnose diabetes and manage it to prevent it from developing into complications, systematic glucose measurement and treatment must be carried out together.

For diabetic patients and people who have not developed diabetes but have more sugar than normal detected in their blood, many medical device manufacturers provide various types of glucose meters that may measure glucose.

There are two types of glucose meters: one that measures blood glucose levels on a one-time basis by drawing blood from the user's fingertip, and one that measures blood glucose levels continuously by attaching the meter to the user's stomach or arm.

In the case of diabetic patients, they generally go back and forth between hyperglycemia and hypoglycemia, and emergency situations occur during hypoglycemia, and loss of consciousness or prolonged hypoglycemia without sugar supply may result in death. Therefore, immediate detection of hypoglycemia is very important for diabetic patients, but glucose meters that measure glucose intermittently have limitations in accurately detecting this condition.

Recently, to overcome these limitations, a continuous glucose monitoring system (CGMS), which is inserted into the human body and measures glucose levels at intervals of several minutes, has been developed and used. In order to minimize the user's pain and resistance following blood collection, the CGMS may measure glucose continuously after inserting a needle-shaped transdermal sensor into areas where pain is relatively less, such as the stomach, arm, etc.

The CGMS includes a sensor transmitter that is inserted into the user's skin to measure glucose in the body and transmit the measured glucose level, and a terminal that outputs the received glucose level.

The terminal may send various notifications to the user when outputting the glucose levels. Notifications may occur according to conditions related to changes in glucose levels, the usage environment of the sensor transmitter, etc. The user may set the conditions for the notifications to ring or the method of outputting the notifications (for example, sound or vibration, frequency of notification, etc.) through the terminal, and may receive notifications according to the settings.

Basically, the terminal may provide the user with a notification about the signal loss with the sensor transmitter. The signal loss may mean a state in which the terminal cannot receive data from the sensor transmitter. In addition, since the notification informs the user of an important situation in glucose management, it is important to provide it through accurate determination in a timely manner. However, if the notification sounds too frequently, the user may be disturbed by the notification in daily life, and on the contrary, if the notification sounds passively, the user may have difficulty noticing the notification. Therefore, in order to provide a notification of signal loss in the CGMS, it is necessary to accurately identify the occurrence of signal loss and output a notification accordingly in a timely manner.

### SUMMARY OF THE INVENTION

Against this background, one object of embodiments of the present disclosure is to accurately determine the occurrence of signal loss and provide a notification of signal loss in a timely manner.

Against this background, another object of embodiments of the present disclosure is to provide a notification of signal loss after a specific period of time during which notification is deferred in order to determine the signal loss.

The present invention is defined by by the method claim 1.

Further aspects of the present invention are outlined in the dependent claims. In order to achieve the above described objects, the invention provides a method of providing a notification of signal loss in a glucose monitoring system including: when a communication failure occurs between a terminal and a sensor transmitter, delaying a signal loss notification for a first period of time by the terminal; when the communication failure persists for the first period of time, further determining whether signal loss persists for a second period of time by the terminal; when the signal loss persists for the second period of time, generating the signal loss notification by the terminal; and outputting, by the terminal, the signal loss notification to a user.

The method may further include delaying the signal loss notification for the second period of time, and the generating of the signal loss notification may include generating the signal loss notification based on a result of determining whether the signal loss persists for the second period of time.

In the method, the determining of whether the signal loss persists for the second period of time may include determining persistence of the signal loss based on data reception of the terminal from the sensor transmitter for the second period of time, and the generating of the signal loss notification may include generating the signal loss notification if the data reception of the terminal from the sensor transmitter is stopped for the second period of time.

The method may further include determining whether the communication failure persists for the first period of time.

In the method, the determining of whether the communication failure persists for the first period of time may include determining persistence of the communication failure based on the communication failure including a case where a communication connection between the terminal and the sensor transmitter is lost or a case where data reception of the terminal from the sensor transmitter is stopped while the communication connection is established.

In the method, the first period of time may be shorter than the second period of time.

In the method, the delaying of the signal loss notification for the first period of time may include outputting biometric information during the first period of time for which the signal loss notification is delayed.

Another embodiment may provide a method of providing a notification of signal loss by a terminal configured to receive biometric information from a sensor transmitter attached to a body, the method including: receiving an advertisement from the sensor transmitter; establishing a communication connection with the sensor transmitter; requesting the biometric information from the sensor transmitter in response to the advertisement; receiving the biometric information from the sensor transmitter; when a communication failure occurs between the terminal and the sensor transmitter, delaying a signal loss notification for a first period of time by the terminal; when the communication failure persists for the first period of time, further determining whether signal loss persists for a second period of time by the terminal; when the signal loss persists for the second period of time, generating the signal loss notification by the terminal; and outputting, by the terminal, the signal loss notification to a user.

In the method, the advertisement may be transmitted from the sensor transmitter to the terminal at each advertisement timing over the first period of time and the second period of time, and the first period of time may include a smaller number of advertisement timings than advertisement timings included in the second period of time.

Another embodiment may provide a method of providing a notification of signal loss by a terminal in a glucose monitoring system, the method including: receiving biometric information from a sensor transmitter attached to a body; outputting the biometric information; when a communication failure occurs between the terminal and the sensor transmitter, delaying a signal loss notification; outputting at least one biometric information while delaying the signal loss notification; after outputting the at least one biometric information, determining whether signal loss persists for a specific period of time; when the signal loss persists for the specific period of time, generating the signal loss notification; and outputting the signal loss notification to a user.

As described above, according to the embodiments, by waiting for a specific period of time to defer notification and simultaneously determining whether signal loss occurs, it is possible to accurately determine the occurrence of signal loss and provide a notification to the user in a timely manner.

### BRIEF DESCRIPTION OF THE DRAWINGS

FIG. 1 is a diagram for schematically explaining a glucose monitoring system according to an embodiment.
FIG. 2 is a diagram for explaining an applicator for attaching a sensor transmitter to a human body according to an embodiment.
FIG. 3 is a diagram for explaining a process of attaching a sensor transmitter to a human body using an applicator according to an embodiment.
FIG. 4 is a configuration diagram of a sensor transmitter according to an embodiment.
FIG. 5 is a configuration diagram of a terminal according to an embodiment.
FIG. 6 is an example diagram in which biometric information is generated in a sensor transmitter or a terminal according to an embodiment.
FIG. 7 an example diagram in which a data packet is generated in a sensor transmitter or a terminal according to an embodiment.
FIG. 8 is a flowchart for explaining a method of transmitting and receiving biometric information between a sensor transmitter and a terminal according to an embodiment.
FIG. 9 is a diagram for explaining an example of a terminal providing a notification of signal loss according to an embodiment.
FIG. 10 is a flowchart for explaining a method of providing a notification of signal loss by a terminal according to an embodiment.
FIG. 11 is a flowchart for specifically explaining a method of providing a notification of signal loss by a terminal according to an embodiment.
FIG. 12 is a diagram for explaining a method of providing a notification of signal loss by a terminal according to another embodiment.
FIG. 13 is a diagram for explaining a method of providing a notification of signal loss by a terminal according to yet another embodiment.

### DETAILED DESCRIPTION

In describing the present disclosure, if it is determined that related known functions may unnecessarily obscure the gist of the present disclosure as they are obvious to those skilled in the art, detailed descriptions thereof will be omitted.

The terms used in the present application are used merely to describe particular embodiments and are not intended to limit the present disclosure. Singular expressions include plural expressions unless the context clearly indicates otherwise. In the present application, it should be understood that terms such as "comprise", "include", or "have" are intended to designate the presence of features, numbers, steps, operations, components, parts, or combinations thereof described in the specification, and they do not preclude the possibility of the presence or addition of one or more other features, numbers, steps, operations, components, parts, or combinations thereof.

Terms such as first and second are merely identifiers used to distinguish identical or corresponding components, and the identical or corresponding components are not limited by terms such as first or second.

Hereinafter, embodiments according to the present disclosure will be described in detail with reference to the accompanying drawings, and in describing with reference to the accompanying drawings, identical or corresponding components will be assigned the same drawing numbers and overlapping descriptions thereof will be omitted.

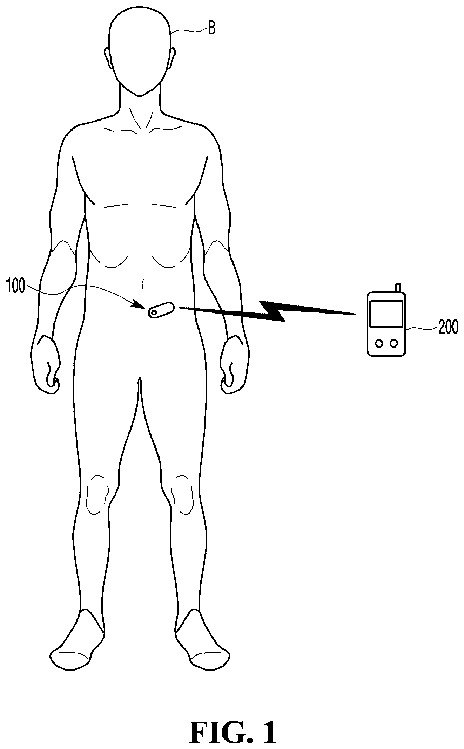

FIG. 1 is a diagram for schematically explaining a glucose monitoring system according to an embodiment.

Referring to FIG. 1, a glucose monitoring system 10 (hereinafter referred to as a "system") according to an embodiment may include a sensor transmitter 100 and a terminal 200.

The sensor transmitter 100 is attached to a human body B, and when the sensor transmitter 100 is attached to the human body B, one end of a sensor of the sensor transmitter 100 is inserted into the skin to periodically extract body fluids from the human body and measure glucose.

The terminal 200 may receive a biosignal including glucose information from the sensor transmitter 100, generate glucose information from the biosignal, and output to the user. The terminal 200 may include, but is not limited to, various devices such as smartphones, mobile phones, tablet PCs, desktops, and laptops, and may have a communication interface capable of communicating with the sensor transmitter 100 and may include a device on which a program or application may be installed.

The sensor transmitter 100 may periodically transmit measured biosignals to the terminal 200 at the request of the terminal 200 or at a set time. For data communication between the sensor transmitter 100 and the terminal 200, the sensor transmitter 100 and the terminal 200 may be connected to each other via a wired connection such as a USB cable or wirelessly using methods such as infrared communication, NFC communication, or Bluetooth.

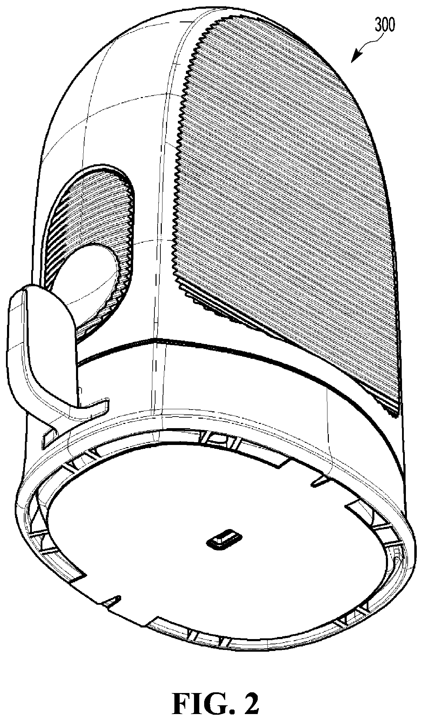

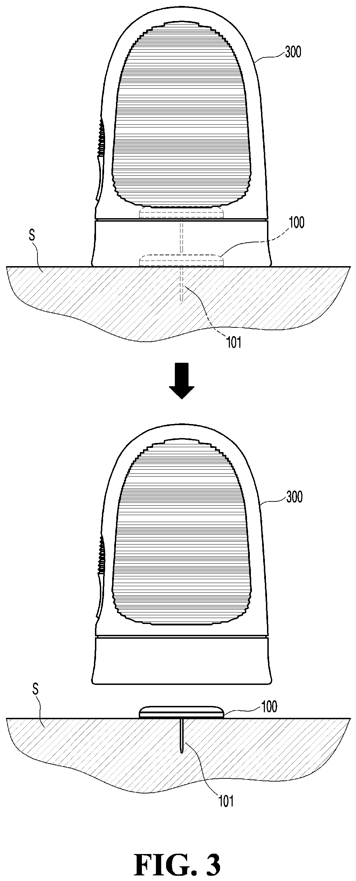

FIG. 2 is a diagram for explaining an applicator for attaching a sensor transmitter to a human body according to an embodiment, and FIG. 3 is a diagram for explaining a process of attaching a sensor transmitter to a human body using an applicator according to an embodiment.

Referring to FIGS. 2 and 3, an applicator 300 according to an embodiment has the sensor transmitter 100 inside and operates to discharge the sensor transmitter 100 to the outside and attach it to a specific body part of the user through manipulation by the user. The applicator 300 is formed in a shape with one side open, and the sensor transmitter 100 is installed in the applicator 300 through the open side of the applicator 300.

When attaching the sensor transmitter 100 to a part of the body using the applicator 300, in order to insert one end of a sensor provided in the sensor transmitter 100 into the skin, the applicator 300 may include a needle (not shown) formed to surround one end of the sensor inside, a first elastic member (not shown) that pushes the needle and one end of the sensor together into the skin, and a second elastic member (not shown) for pulling out only the needle. Through this configuration of the applicator 300, the needle and one end of the sensor may be simultaneously inserted into the skin by decompressing the first elastic member (not shown) disposed in a compressed state inside the applicator 300. When one end of the sensor is inserted into the skin, only the needle is pulled out by decompressing the compressed second elastic member (not shown). The user may safely and easily attach the sensor transmitter 100 to the skin through the applicator 300.

Looking in detail at the process of attaching the applicator 300 to the human body B, with a protective cap (not shown) removed, the open side of the applicator 300 is brought into close contact with the skin S of a specific area of the human body B. When the applicator 300 is operated in this way while the applicator 300 is in close contact with the skin S of the human body B, the sensor transmitter 100 is discharged from the applicator 300 and may be attached to the skin S. Here, one end of the sensor 101 is disposed at a lower part of the sensor transmitter 100, exposed from the sensor transmitter 100, and one end of the sensor 101 may be partially inserted into the skin S through the needle provided in the applicator 300. Therefore, the sensor transmitter 100 may be attached to the skin S with one end of the sensor 101 inserted into the skin S.

Here, an adhesive tape may be provided on the surface of the sensor transmitter 100 in contact with the human body B so that the sensor transmitter 100 may be fixedly attached to the skin S of the human body B. Therefore, when the applicator 300 is separated from the skin S of the human body B, the sensor transmitter 100 may be fixedly attached to the skin S of the human body B by the adhesive tape.

Afterwards, when power is applied to the sensor transmitter 100, the sensor transmitter 100 communicates with the terminal, and the sensor transmitter 100 may transmit biosignals including glucose information to the terminal. The sensor transmitter 100 may generate not only glucose information but also various biometric information, and hereinafter, it will be explained that glucose information is measured as an example of biometric information.

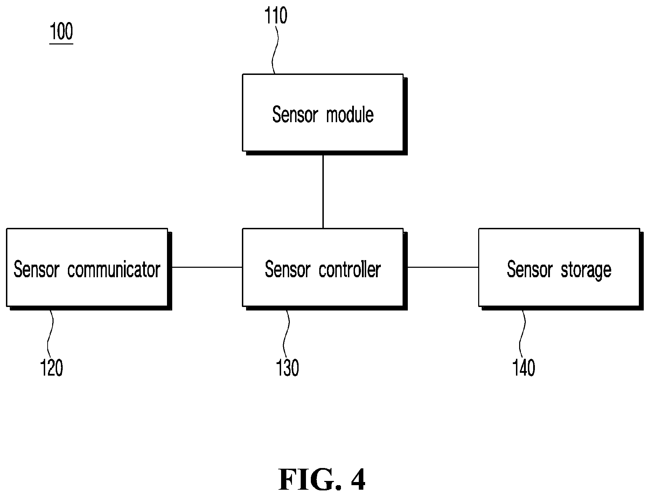

FIG. 4 is a configuration diagram of a sensor transmitter according to an embodiment.

Referring to FIG. 4, the sensor transmitter 100 according to an embodiment may include a sensor module 110, a sensor communicator 120, a sensor controller 130, and a sensor storage 140.

The sensor module 110 may include at least one sensor that is inserted into the human body and senses biomass. At least one sensor may measure biomass and generate biosignals. The biosignal is an analog signal and may include a current value.

The sensor communicator 120 may exchange data or information with the terminal. For example, the sensor communicator 120 may transmit biosignals received from the sensor module 110 or data stored in the sensor storage 140 (for example, biometric information) to the terminal.

The sensor controller 130 may control the overall configuration of the sensor transmitter 100, including the sensor module 110, the sensor storage 140, and the sensor communicator 120. For example, the sensor controller 130 may receive a control signal from the terminal and control the configuration of the sensor transmitter 100 accordingly. In addition, the sensor controller 130 may process biosignals. For example, the sensor controller 130 may convert biosignals into analog or digital form or perform processing to remove noise as needed.

Data or information may be stored in the sensor storage 140. For example, the sensor storage 140 may store data on biomass measured by the sensor module 110, for example, the current value of the biosignal or its digital form data, or data received from the terminal, for example, the command value of the control signal.

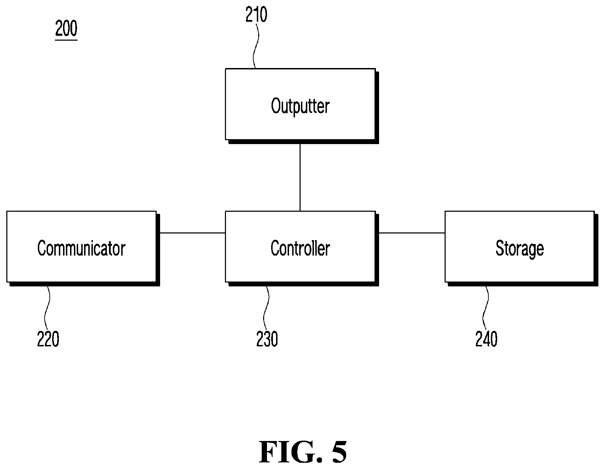

FIG. 5 is a configuration diagram of a terminal according to an embodiment.

Referring to FIG. 5, the terminal 200 according to an embodiment may include an outputter 210, a communicator 220, a controller 230, and a storage 240.

The outputter 210 may output biometric information included in the biosignal, for example, glucose information, so that the user may check it. For example, the outputter 210 may display glucose information as numerical levels (values) or a graph processed from the numerical levels (values).

The communicator 220 may communicate with the sensor communicator of the sensor transmitter and exchange data or information. For example, the communicator 220 may receive a biosignal containing information (biometric information) about biomass measured by the sensor transmitter. Here, the communicator 220 may receive primarily processed biosignals from the sensor transmitter. Preferably, the processed biosignal may include discrete data (discontinuous data) in which a current value, which is an analog signal, is converted into digital data. If the current value is sampled every cycle, digital discrete data may be generated. Alternatively, the communicator 220 may transmit a control signal for controlling the sensor transmitter to the sensor transmitter.

Data or information may be stored in the storage 240. For example, data received from the sensor transmitter, for example, biometric information, may be stored in the storage 240. Here, biometric information may include digital data representing current values as glucose information. Alternatively, data input from the user or environment setting data for setting the operating environment of the terminal may be stored in the storage 240.

The controller 230 may include at least one processor that executes a program that provides notification of signal loss in a glucose monitoring system and at least one memory in which the program is stored. The memory and processor included in the controller 230 may be integrated into one chip or may be physically separated.

Memory may be implemented as non-volatile memory devices such as read only memory (ROM), programmable ROM (PROM), erasable programmable ROM (EPROM), electrically erasable programmable ROM (EEPROM), and flash memory, or volatile memory devices such as random access memory (RAM) to store various programs, data, and/or information.

In addition, the controller 230 may generate a glucose value, which is quantified glucose information, from biometric information. To this end, the controller 230 may obtain biometric information in the form of a current value from the sensor transmitter, and preprocess and/or process the current value of the biometric information. The controller 230 may first calculate the sensitivity and generate the glucose value according to this sensitivity.

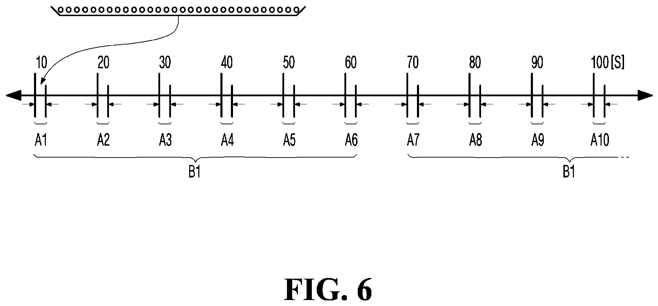

FIG. 6 is a diagram illustrating a first example of generating biometric information in a sensor transmitter according to an embodiment.

Referring to FIG. 6, biometric information may be generated in a sensor transmitter according to an embodiment. Specifically, the sensor transmitter may obtain an analog (continuous) biosignal representing a current value at predetermined intervals, and sample the biosignal to generate digital (discontinuous) data representing the current value. The generated data may be processed in the sensor transmitter to generate biometric information. Hereinafter, it is described that biometric information is generated by generating and processing digital data from a biosignal in the sensor transmitter, but is not limited thereto, and part or all of the processing may be performed in the sensor transmitter depending on the embodiment.

For example, the sensor transmitter may obtain a biosignal in an analog form, for example, a current value, measure the biosignal every 10 seconds, and process the measured biosignal to generate a single piece of first data. Specifically, the sensor transmitter may measure (sample) a biosignal 30 times every 10 seconds and generate digital data. The sensor transmitter may remove upper data and lower data from the 30 pieces of data, calculate an average value (A1) of the remaining data, and determine this average value (A1) as the single first data. The first data, which is the data of the average value (A1) calculated in this way, is generated in 10 second-units, and as illustrated, six average values (A1 to A6), i.e., six pieces of first data, may be generated in 1 minute.

In addition, the sensor transmitter may process 30 pieces of first data every 300 seconds (5 minutes).

The sensor transmitter may generate an average value (B1) again using the six pieces of first data (average values (A1 to A6)). In generating the average value (B1), the terminal may remove the upper data and lower data among the six average values (A1 to A6) and generate the average value (B1) of the remaining data. Second data, which is the data of the average value (B1) calculated in this way, is generated in 1 minute-units, and as illustrated, one average value (B1), i.e., one second data, may be generated in 1 minute.

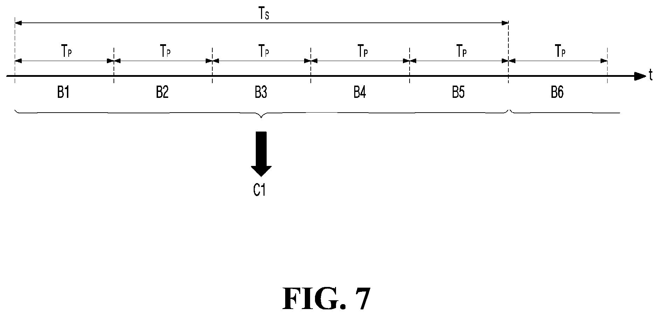

FIG. 7 is a diagram illustrating a second example of generating biometric information in a sensor transmitter according to an embodiment.

Referring to FIG. 7, second data may be processed to generate biometric information in a sensor transmitter according to an embodiment. In the above-described example, the sensor transmitter may obtain 5 pieces of second data (B1) in 1 minute-units from 6 pieces of first data in 10 second-units. In addition, the sensor transmitter may generate an average value (C1) using the 5 pieces of second data (average values (B1 to B5)). In generating the average value (C1), the terminal may remove upper data and lower data among the 5 average values (B1 to B5) and generate an average value (C1) of the remaining data. Third data, which is the data of the average value (C1) calculated in this way, is generated in 5-minute units, and as illustrated, one average value (C1), i.e., one third data, may be generated in 5 minutes.

Here, the terminal may sequentially generate second data (B1 to B5) during a biometric information generation period (Tp) and generate one third data (C1) during a glucose value biometric information generation period (Ts). In the glucose value biometric information generation period (Ts), a glucose value is calculated from the third data (C1) through sensitivity, and in the biometric information generation period (Tp), second data (B1 to B5) that serve as the basis for the third data (C1) may be generated. In the example described above, the biometric information generation period (Tp) may correspond to 1 minute, and the glucose value biometric information generation period (Ts) may correspond to 5 minutes.

In this way, the third data generated in 5-minute units may undergo a noise removal process by a filter. The filtered third data may be converted into biometric information including a glucose value by applying sensitivity, and such biometric information may be output to the user.

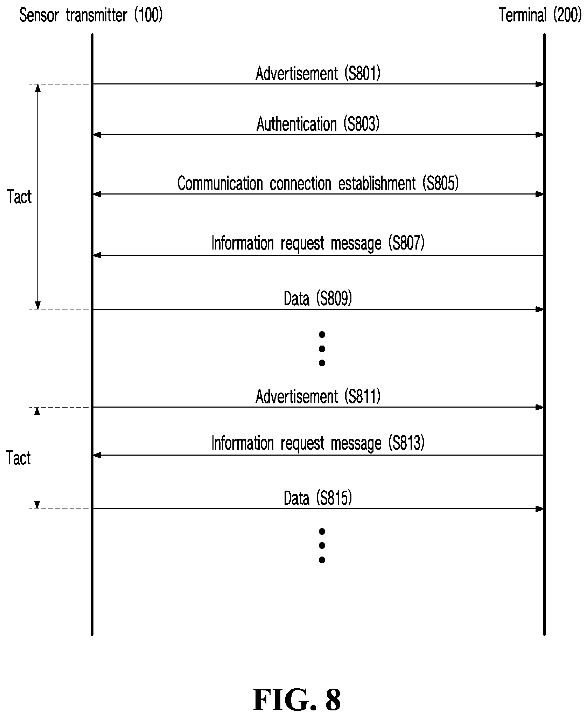

FIG. 8 is a flowchart for explaining a method of transmitting and receiving biometric information between a sensor transmitter and a terminal according to an embodiment.

Referring to FIG. 8, the sensor transmitter 100 and the terminal 200 according to an embodiment may connect communication in order to transmit and receive data including biometric information. Data may be transmitted and received after the communication connection. The sensor transmitter 100 and the terminal 200 may be connected to each other through wired or wireless communication, and may connect communication in a manner such as USB communication, infrared communication, Bluetooth communication, etc.

Specifically, the sensor transmitter 100 and the terminal 200 may transmit and receive differently depending on whether communication is disconnected and a new communication is connected (when connecting initial communication) or only data is transmitted and received after initial communication is connected. First, in a state of communication disconnection, when the sensor transmitter 100 and the terminal 200 establish a new communication connection, the sensor transmitter 100 may advertise to the terminal 200 (step S801). The sensor transmitter 100 may generate a certain signal for advertisement and transmit this advertisement signal to the terminal. Alternatively, the sensor transmitter 100 may perform advertisement by periodically transmitting an advertisement message to the terminal 200. The process of sending this advertisement signal or advertisement message may be called advertisement. The terminal 200 may receive the advertisement signal or the advertisement message and perform authentication with the sensor transmitter 100 (step S803). For example, the sensor transmitter 100 and the terminal 200 may authenticate that they are valid devices through a hash value. Then, the sensor transmitter 100 and the terminal 200 may establish a communication connection (step S805). The communication connection is a state in which data may be transmitted and received immediately without going through a separate initial communication process such as authentication, and the sensor transmitter 100 and the terminal 200 may transmit and receive data in response to an advertisement at any time while the communication connection is being established. The terminal 200 may transmit an information request message to the sensor transmitter 100 to obtain data including biometric information (for example, 30 pieces of first data) (step S807). The sensor transmitter 100 that has received the information request signal or information request message may transmit data including biometric information (for example, 30 pieces of first data) to the terminal 200 in response thereto (step S809).

Once the initial communication is connected, data may be transmitted and received in the communication connection establishment state without a separate authentication process. The sensor transmitter 100 may advertise an advertisement message to the terminal 200 repeatedly, periodically or aperiodically, during the following operation section (step S811). The repeated advertisement may be performed by transmitting an advertisement signal or an advertisement message to the terminal 200 periodically or aperiodically. The terminal 200 may send an information request message requesting data transmission in response to the advertisement to the sensor transmitter 100 (step S813). The sensor transmitter 100 may transmit data in response to the information request signal or information request message (step S815).

Here, the terminal 200 receives data from the sensor transmitter 100 from time to time, but the terminal 200 may receive data in response to the advertisement only when the sensor transmitter 100 advertises. The sensor transmitter 100 may send this advertisement signal or advertisement message to the terminal 200 periodically or aperiodically, and the terminal 200 may request and receive data accordingly. In addition, the sensor transmitter 100 may not continuously send the advertisement signal or advertisement message, but may transmit only in active mode, i.e., while awake. Accordingly, data transmission and reception between the sensor transmitter 100 and the terminal 200 may be performed only during this active mode period (Tact). The sensor transmitter 100 may stand by without sending any data during the inactive mode, i.e, the non-active mode period.

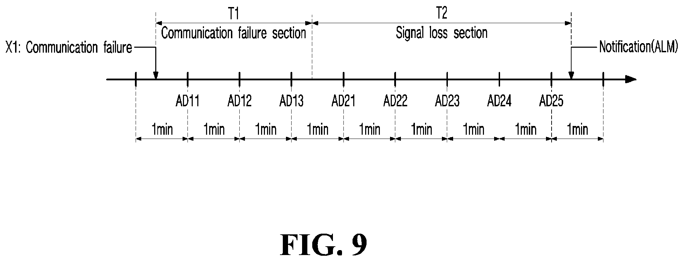

FIG. 9 is a diagram for explaining an example of a terminal providing a notification of signal loss according to an embodiment.

Referring to FIG. 9, an example of a terminal providing a notification of signal loss according to an embodiment may be illustrated. The terminal may provide a notification to a user under various conditions. In particular, in the present disclosure, a method of providing a notification by a terminal when a communication failure and resulting signal loss occur is explained, and the method will be described in detail below.

Here, the communication failure may mean a state in which data cannot be received due to a communication module (e.g., a Bluetooth communication module) being turned off or due to any failure (e.g., when the distance between the sensor transmitter and the terminal increases) even when the communication module is turned on. The former example corresponds to a case in which the communication connection between the sensor transmitter and the terminal is cut off, and the latter example corresponds to a case in which the terminal stops receiving data from the sensor transmitter while the communication connection is established. Further, signal loss may mean a state in which data cannot be received, regardless of a failure of the communication module. Communication failure considers both the operating status of the communication module and data non-reception, but signal loss considers only data non-reception without considering the communication module, so the two may be conceptually different. The terminal may determine whether there is a communication failure based on the operating status of the communication module and whether data is receive, and may determine the presence of signal loss through whether data is received.

Specifically, a communication failure may occur at time X1 while the sensor transmitter and the terminal are communicating. The terminal may detect whether a communication failure has occurred through the communicator. In addition, the terminal may wait for a first period of time (T1) without sounding a notification for signal loss, i.e., a signal loss notification (ALM). The communication module may be turned-off, or the terminal may not receive data even if the communication module is turned on. Therefore, even if the sensor transmitter transmits a signal or message to the terminal for advertisement, the terminal may not receive the signal or message. Alternatively, even if the signal or message reaches the terminal, the terminal may not be able to transmit the information request message or receive data including biometric information in response to the information request message. At the time point when the sensor transmitter advertises, i.e., at the advertisement timing (AD11, AD12, AD13), each terminal may not be able to receive data from the sensor transmitter as described above.

If no communication failure occurs, the advertisement timing (AD11, AD12, AD13) may, in principle, include the following characteristics. The advertisement timings (AD11, AD12, AD13) may be periodic or aperiodic. When the advertisement timings (AD11, AD12, AD13) are periodic, the advertisement timings (AD11, AD12, AD13) may be formed at 1-minute intervals. At the advertisement timings (AD11, AD12, AD13), i.e., at 1-minute intervals, the sensor transmitter may advertise to the terminal and send a signal or message for this advertisement to the terminal. In addition to the advertisement, the sensor transmitter may transmit data including biometric information to the terminal at the advertisement timings (AD11, AD12, AD13). Strictly speaking, when the sensor transmitter collects data from the sensor for a predetermined period of time, the collected data may be transmitted to the terminal at any one of the advertisement timings (AD11, AD12, AD13). As in the example described above, the sensor transmitter may collect and process 300 seconds (5 minutes) of data and transmit to the terminal. The sensor transmitter may continuously collect first data in units of 10 seconds, generate second data in units of 60 seconds (1 minute) from the first data, and generate third data in units of 300 seconds (5 minutes) from the second data. When 300 seconds (5 minutes) of data (i.e., the third data) are completed at any one of the advertisement timings (AD11, AD12, AD13), the sensor transmitter may send the 300 seconds (5 minutes) of data to the terminal at once. For example, if the sensor transmitter has already completed 300 seconds (5 minutes) of data at the advertisement timing (AD11), it may only advertise and not transmit data at the advertisement timing (AD12, AD13). If the sensor transmitter should have completed 300 seconds (5 minutes) of data at the advertisement timing (AD11) but did not, it may complete 300 seconds (5 minutes) of data by the next advertisement timing (AD12 or AD13) and transmit it to the terminal.

Advertisement timings (AD11, AD12, AD13) are repeated, and advertisement timings (AD21, AD22, AD23, AD24, AD25) may also be formed at 1-minute intervals thereafter. If the sensor transmitter completes 300 seconds (5 minutes) of data at advertisement timing (AD13) and sends it to the terminal, the sensor transmitter may transmit data to the terminal again at advertisement timing (AD25), which is 300 seconds (5 minutes) later. At other advertisement timings (AD21, AD22, AD23, AD24), the sensor transmitter may only advertise without transmitting data. Here, the advertisement timings (AD11, AD12, AD13) included in the first period of time (T1) may be named as the first advertisement timing, and the advertisement timings (AD21, AD22, AD23, AD24, AD25) included in the second period of time (T2) may be named as the second advertisement timing, respectively.

When a communication failure occurs, the terminal may immediately delay the generation and provision of a notification of a signal loss notification (ALM) and wait. The terminal may continue to delay the notification while data reception is stopped due to the communication failure and wait. For example, if the terminal does not receive an advertisement and data from the sensor transmitter during the first period of time (T1), i.e., all of the first advertisement timings (AD11, AD12, AD13), the terminal may delay the signal loss notification (ALM) and wait during the first period of time (T1). Then, when the first period of time (T1) has elapsed, the terminal may detect through the communicator whether the signal loss persists for the second period of time (T2). The terminal may still not receive advertisements and data from the sensor transmitter during all of the second advertisement timings (AD21, AD22, AD23, AD24, AD25). If the signal loss persists for the second period of time (T2), the terminal may further delay the signal loss notification (ALM) for the second period of time (T2) and maintain the standby operation of the first period of time (T1). Rather than sounding the signal loss notification (ALM) because the first period of time (T1) has elapsed, the terminal may further determine whether the signal loss persists for the second period of time (T2).

If the signal loss still persists after the second period of time (T2) has elapsed, the terminal may then generate the signal loss notification (ALM). And the terminal may provide the signal loss notification (ALM) to the user through the outputter.

Here, the first period of time (T1) may be preset and may vary depending on how many of the first advertisement timings (AD11, AD12, AD13) are included. The more advertisement timings there are, the longer the first period of time (T1) may be. In addition, the second period of time (T2) may also be preset and may vary depending on how many of the second advertisement timings (AD21, AD22, AD23, AD24, AD25) are included. The more advertisement timings there are, the longer the second period of time (T2) may be. Preferably, the first period of time (T1) may be shorter than the second period of time (T2), and thus may include fewer advertisement timings. And in the present drawing, the first period of time (T1) is set to 3 minutes and the second period of time (T2) is set to 5 minutes, and each includes the corresponding first advertisement timings (AD11, AD12, AD13) and second advertisement timings (AD21, AD22, AD23, AD24, AD25), but may be set longer than this. For example, the first period of time (T1) may be set to 15 minutes and the second period of time (T2) may be set to 25 minutes, respectively. The first period of time (T1) includes 15 advertisement timings and the second period of time (T2) includes 25 advertisement timings.

In addition, since the first period of time (T1) experiences signal loss due to a communication failure that occurred at time X1, it may also be named a 'communication failure section.' On the other hand, the second period of time (T2) is a region in which it is determined whether signal loss persists further following the first period of time (T1), and therefore, it may be named a 'signal loss section' to be distinguished independently from the first period of time (T1).

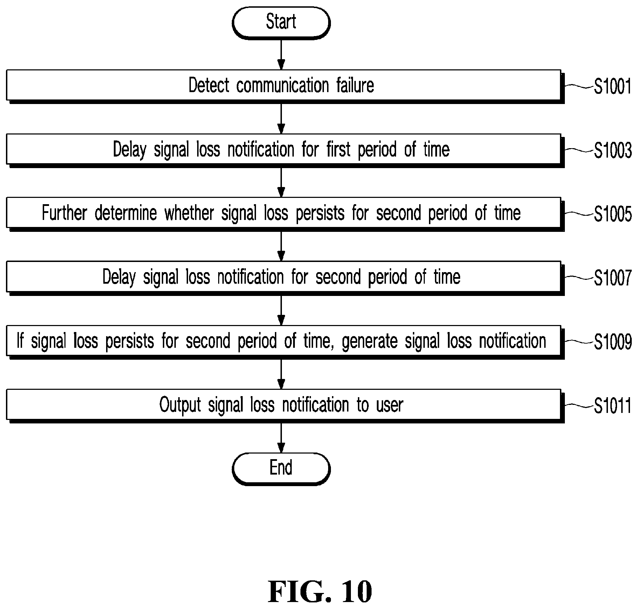

FIG. 10 is a flowchart for explaining a method of providing a notification of signal loss by a terminal according to an embodiment.

Referring to FIG. 10, a method for providing a signal loss notification of a terminal according to an embodiment may be illustrated. The terminal may detect a communication failure, provide a signal loss notification after determining whether the signal loss persists after providing a plurality of grace periods for the signal loss notification.

The controller of the terminal may detect a communication failure in a relationship with the sensor transmitter through the communicator (step S1001).

For the first period of time, the controller of the terminal may determine whether the signal loss due to the communication failure, i.e., the state in which data is not continuously received, persists. If the signal loss persists for the first period of time, the controller of the terminal may delay the signal loss notification (step S1003). Meanwhile, even while the signal loss notification is delayed for the first period of time, the controller of the terminal may control the outputter to output biometric information. Even before the communication failure, the outputter of the terminal may display biometric information in the form of a glucose value to the user, and even after the communication failure, the outputter of the terminal may maintain the output of the biometric information as it is for the first period of time.

Then, after the first period of time has elapsed, the controller of the terminal may determine whether the signal loss still persists for the second period of time (step S1005). If the signal loss still persists for the second period of time, the controller of the terminal may further delay the signal loss notification for the second period of time (step S1007). If the second period of time has elapsed and the signal loss still persists for the second period of time, the controller of the terminal may generate the signal loss notification (step S1009). The outputter of the terminal may output the generated signal loss notification to the user (step S1011).

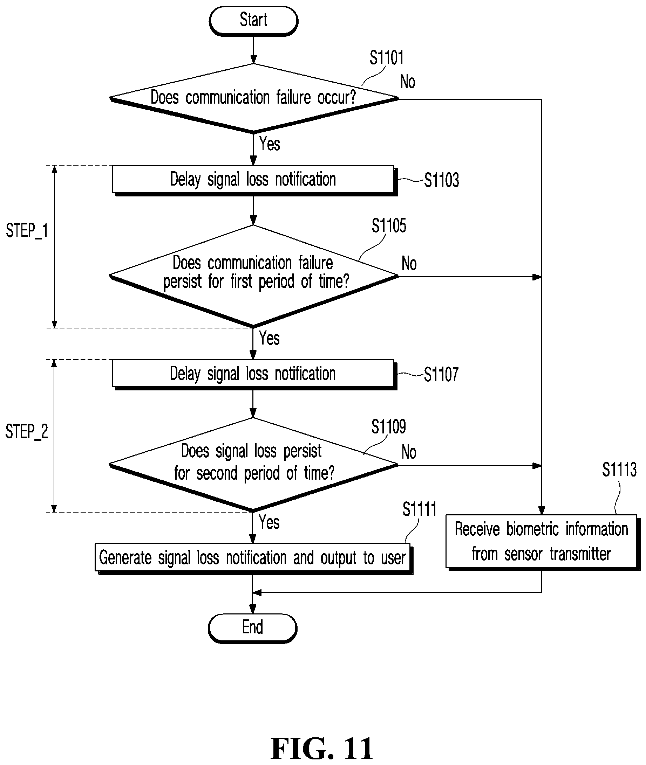

FIG. 11 is a flowchart for specifically explaining a method of providing a notification of signal loss by a terminal according to an embodiment.

Referring to FIG. 11, a method for providing a signal loss notification of a terminal according to an embodiment may be illustrated in detail. The terminal may delay the signal loss notification for a plurality of grace periods in order to generate and output the signal loss notification. During this time, the terminal may determine whether a signal loss notification should be provided, and these determinations may be performed during the plurality of grace periods. During the first period of time, the terminal may make a determination on whether to additionally secure a second grace period, i.e., first determination. In addition, during the wait of the second period of time, the terminal may make a determination on whether to terminate the grace period and generate a signal loss notification, i.e., second determination. In the present drawing, the first determination performed by the terminal during the first period of time and the second determination performed during the second period of time will be described.

The controller of the terminal may determine whether a communication failure has occurred (step S1101). If it is determined that a communication failure has not occurred, the terminal may receive biometric information from the sensor transmitter at the advertisement timing (NO of step S1101 and S1113).

If it is determined that a communication failure has occurred, the terminal may perform a first step (STEP1) including the first determination during the first period of time. First, the controller of the terminal may delay the signal loss notification (step S1103). Then, the controller of the terminal may perform the first determination on whether to add the second period of time. This may mean that since the second determination on whether to generate the signal loss notification is performed in the second period of time, the first determination is a premise for determining whether to start the second determination. Therefore, the controller of the terminal may determine whether the communication failure persists even while waiting for the first period of time (the first determination, step S1105). If it is determined that the communication failure does not persist, the terminal may receive biometric information from the sensor transmitter at the advertisement timing (NO of step S1105 and S1117).

If it is determined that the communication failure persists, the terminal may perform a second step (STEP2) including the second determination during the second period of time. First, the controller of the terminal may delay the signal loss notification (YES of step S1105 and step S1107). Then, the controller of the terminal may perform the second determination on whether to generate the signal loss notification. As described above, the second determination may be performed only when it is decided through the first determination that the communication failure persists and the second period of time is to be added. Therefore, the controller of the terminal may determine whether the signal loss persists for the second period of time (the second determination, step S1111). If it is determined that the signal loss does not persist, the terminal may receive biometric information from the sensor transmitter at the advertisement timing (NO of step S1111 and step S1113).

If it is determined that the signal loss persists, the controller of the terminal may generate the signal loss notification and output it to the user (YES of step S1111 and step S1111).

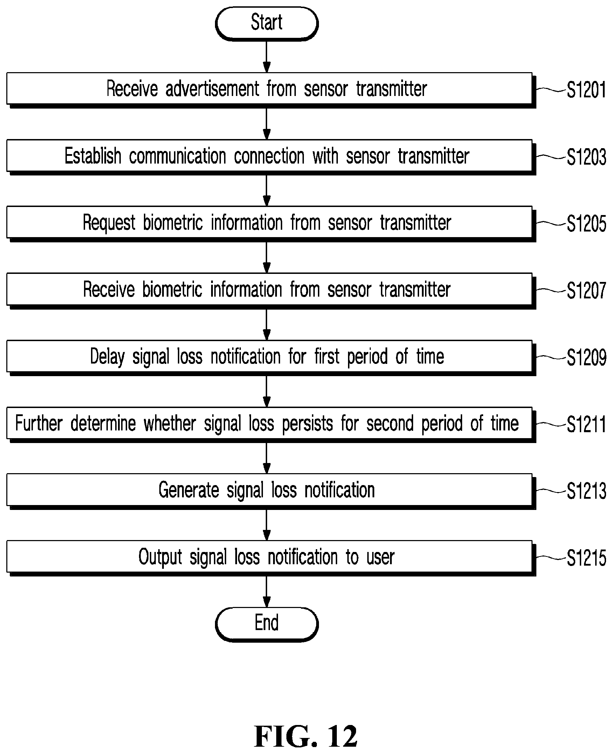

FIG. 12 is a diagram for explaining a method of providing a notification of signal loss by a terminal according to another embodiment.

Referring to FIG. 12, a method of providing a notification of signal loss by a terminal according to another embodiment may be illustrated. The above-described embodiment is also commonly applied to the present embodiment, and the following description will focus on differences.

In the above-described embodiment, a communication failure occurs at any point in time, but in the present embodiment, a communication failure may occur because the terminal fails to receive data at any one of the advertisement timings. The former implies that the communication module is turned off at any point in time, but the latter may imply that the terminal fails to receive data at the advertisement timing regardless of the state of the communication module. In the latter case, the method of providing a signal loss notification by a terminal may be as follows.

The terminal may periodically receive an advertisement (message) from the sensor transmitter (step S1201). The terminal may request and establish a communication connection to the sensor transmitter through an advertisement, and may request biometric information based thereon and receive biometric information from the sensor transmitter (step S1203, step S1205, and step S1207).

During data transmission and reception, the terminal may not receive data at one advertisement timing. The terminal may delay the signal loss notification for the first period of time from one advertisement timing (step S1209). The terminal may determine whether data reception is stopped at all advertisement timings included in the first period of time.

If the stoppage of data reception, i.e., signal loss, persists for the first period of time and the first period of time has elapsed, the terminal may further determine whether the signal loss persists for the second period of time (step S1211). At the same time, the terminal may delay the signal loss notification even during the second period of time. If the signal loss persists for the second period of time and the second period of time has elapsed, the terminal may then generate the signal loss notification (step S1213). In addition, the terminal may output the signal loss notification to the user (step S1215).

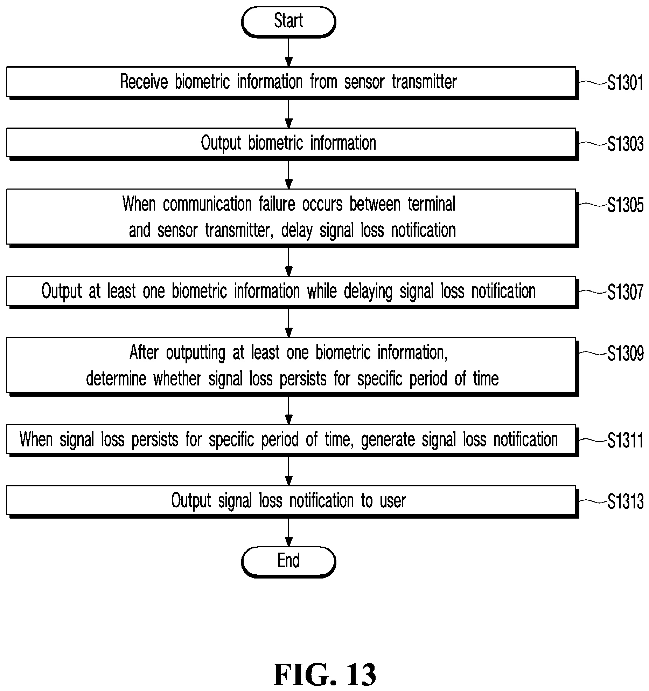

FIG. 13 is a diagram for explaining a method of providing a notification of signal loss by a terminal according to yet another embodiment.

Referring to FIG. 13, a method of providing a notification of signal loss by a terminal according to yet another embodiment may be illustrated. The above-described embodiment is also commonly applied to the present embodiment, and the following description will focus on differences.

In the present embodiment, as in the above-described embodiment, when the communication failure persists for the first period of time and the signal loss persists for the second period of time, the terminal may delay a signal loss notification. However, the terminal may output at least one biometric information even during the first period of time. Despite the communication failure occurring during the first period of time, the terminal may output at least one biometric information. The period of time during which at least one biometric information is output may correspond to the first period of time.

The terminal may receive biometric information from the sensor transmitter and output the biometric information to the user (step S1301 and step S1303). Meanwhile, if a communication failure occurs between the terminal and the sensor transmitter, the terminal may delay without outputting a signal loss notification (S1305 step). The terminal may output at least one biometric information even while delaying the signal loss notification (step S1307). Even if a communication failure occurs and persists, the terminal outputs the biometric information.

After outputting at least one biometric information, the terminal may determine whether the signal loss persists for a specific period of time (step S1309). If the signal loss persists for the specific period of time, the terminal may generate a signal loss notification (step S1311). Here, the specific period of time is a period of time granted to the first period of time during which at least one biometric information is output, and thus may correspond to the second period of time. In addition, the terminal may output the signal loss notification to the user (step S1313).

The aspects of the subject matter described herein may be described in the context of computer-executable instructions, such as program modules, that are executed on a computer. Generally, program modules include routines, programs, objects, components, data structures, etc., which perform specific tasks or specific abstract data types.

Alternatively or additionally, the functionality described herein may be performed, at least in part, by one or more hardware logic components. By way of example, and not limitation, exemplary types of hardware logic components that may be used include field-programmable gate array (FPGA), program-specific integrated circuit (ASIC), application-specific standard product (ASSP), system-on-a-chip system (SOC), complex programmable logic device (CPLD), etc.

In addition, the disclosed embodiments may be implemented in the form of a non-transitory recording medium that stores programs and/or instructions executable by a computer. Instructions may be stored in the form of program code, and when executed by a processor, may create program modules to perform operations of the disclosed embodiments. The non-transitory recording medium may be implemented as a non-transitory computer-readable recording medium.

Computer-readable recording media include all types of recording media storing instructions that may be decoded by a computer. For example, there may be read only memory (ROM), random access memory (RAM), magnetic tape, magnetic disk, flash memory, optical data storage, etc.

Although embodiments of the present disclosure have been described above, those skilled in the art will be able to make various modifications and changes to the present disclosure by adding, changing or deleting components, without departing from the present invention as set forth in the appended claims, which are included within the scope of rights of the present disclosure.

## Claims

1. A method of providing a notification of signal loss in a glucose monitoring system, the method comprising:
when a communication failure occurs between a terminal and a sensor transmitter, delaying (S1003) a signal loss notification for a first period of time by the terminal;
determining (S1105) whether the communication failure persists for the first period of time by the terminal;
when the communication failure persists for the first period of time, delaying (S1107) the signal loss notification for a second period of time followed by the first period of time;
further determining (S1109) whether signal loss persists for the second period of time by the terminal;
when the signal loss persists for the second period of time, generating (S1009) the signal loss notification by the terminal; and
outputting (S1011), by the terminal, the signal loss notification to a user.

2. The method according to claim 1, wherein the generating (S1009) of the signal loss notification comprises generating the signal loss notification based on a result of determining whether the signal loss persists for the second period of time.

3. The method according to claim 2, wherein the determining (S1109) of whether the signal loss persists for the second period of time comprises determining persistence of the signal loss based on data reception of the terminal from the sensor transmitter for the second period of time, and
the generating (S1009) of the signal loss notification comprises generating the signal loss notification if the data reception of the terminal from the sensor transmitter is stopped for the second period of time.

4. The method according to claim 1, wherein the determining (S1105) of whether the communication failure persists for the first period of time comprises determining persistence of the communication failure based on the communication failure including a case where a communication connection between the terminal and the sensor transmitter is lost or a case where data reception of the terminal from the sensor transmitter is stopped while the communication connection is established.

5. The method according to claim 1, wherein the first period of time is shorter than the second period of time.

6. The method according to claim 1, wherein the delaying (S1003) of the signal loss notification for the first period of time comprises outputting biometric information during the first period of time for which the signal loss notification is delayed.

7. The method according to claim 1, further comprising:
receiving (S1201), before the communication failure occurs, an advertisement from the sensor transmitter;
establishing (S1203) a communication connection with the sensor transmitter;
requesting (S1205) the biometric information from the sensor transmitter in response to the advertisement; and
receiving (S1207) the biometric information from the sensor transmitter.

8. The method according to claim 1, wherein the advertisement is transmitted from the sensor transmitter to the terminal at each advertisement timing over the first period of time and the second period of time, and
the first period of time includes a smaller number of advertisement timings than advertisement timings included in the second period of time.

9. The method according to claim 1, further comprising::
receiving (S1301), before the communication failure occurs, biometric information from a sensor transmitter attached to a body;
outputting (S1303) the biometric information; and
outputting (S1307), after the communication failure occurs, at least one biometric information while delaying the signal loss notification.

## Patentansprüche

1. Verfahren zum Bereitstellen einer Benachrichtigung über Signalverlust in einem Glucoseüberwachungssystem, wobei das Verfahren Folgendes umfasst:
wenn eine Kommunikationsstörung zwischen einem Endgerät und einem Sensorsender auftritt, Verzögern (S1003) einer Signalverlustbenachrichtigung für einen ersten Zeitraum durch das Endgerät;
Bestimmen (S1105), ob die Kommunikationsstörung für den ersten Zeitraum andauert, durch das Endgerät;
wenn die Kommunikationsstörung für den ersten Zeitraum andauert, Verzögern (S1107) der Signalverlustbenachrichtigung für einen zweiten Zeitraum, gefolgt von dem ersten Zeitraum;
ferner Bestimmen (S1109), ob der Signalverlust für den zweiten Zeitraum andauert, durch das Endgerät;
wenn der Signalverlust für den zweiten Zeitraum andauert, Erzeugen (S1009) der Signalverlustbenachrichtigung durch das Endgerät; und
Ausgeben (S1011) der Signalverlustbenachrichtigung an einen Benutzer durch das Endgerät.

2. Verfahren nach Anspruch 1, wobei das Erzeugen (S1009) der Signalverlustbenachrichtigung Erzeugen der Signalverlustbenachrichtigung basierend auf einem Ergebnis eines Bestimmens, ob der Signalverlust für den zweiten Zeitraum andauert, umfasst.

3. Verfahren nach Anspruch 2, wobei das Bestimmen (S1109), ob der Signalverlust für den zweiten Zeitraum andauert, Bestimmen eines Andauerns des Signalverlusts basierend auf einem Datenempfang des Endgeräts von dem Sensorsender für den zweiten Zeitraum umfasst und
das Erzeugen (S1009) der Signalverlustbenachrichtigung Erzeugen der Signalverlustbenachrichtigung, falls der Datenempfang des Endgeräts von dem Sensorsender für den zweiten Zeitraum gestoppt ist, umfasst.

4. Verfahren nach Anspruch 1, wobei das Bestimmen (S1105), ob die Kommunikationsstörung für den ersten Zeitraum andauert, Bestimmen eines Andauerns der Kommunikationsstörung basierend darauf, dass die Kommunikationsstörung beinhaltend einen Fall, in dem eine Kommunikationsverbindung zwischen dem Endgerät und dem Sensorsender verloren geht, oder einen Fall, in dem ein Datenempfang des Endgeräts von dem Sensorsender gestoppt ist, während die Kommunikationsverbindung hergestellt ist, umfasst.

5. Verfahren nach Anspruch 1, wobei der erste Zeitraum kürzer als der zweite Zeitraum ist.

6. Verfahren nach Anspruch 1, wobei das Verzögern (S1003) der Signalverlustbenachrichtigung für den ersten Zeitraum Ausgeben biometrischer Informationen während des ersten Zeitraums, für den die Signalverlustbenachrichtigung verzögert ist, umfasst.

7. Verfahren nach Anspruch 1, ferner umfassend:
Empfangen (S1201), bevor die Kommunikationsstörung auftritt, einer Ankündigung von dem Sensorsender;
Herstellen (S1203) einer Kommunikationsverbindung mit dem Sensorsender;
Anfordern (S1205) der biometrischen Informationen von dem Sensorsender als Reaktion auf die Ankündigung; und
Empfangen (S1207) der biometrischen Informationen von dem Sensorsender.

8. Verfahren nach Anspruch 1, wobei die Ankündigung von dem Sensorsender an das Endgerät zu jedem Ankündigungszeitpunkt über den ersten Zeitraum und den zweiten Zeitraum übertragen wird und
der erste Zeitraum eine geringere Anzahl von Ankündigungszeitpunkten als Anzeigenzeitpunkte, die in dem zweiten Zeitraum enthalten sind, enthält.

9. Verfahren nach Anspruch 1, ferner umfassend:
Empfangen (S1301), bevor die Kommunikationsstörung auftritt, biometrischer Informationen von einem Sensorsender, der an einem Körper angebracht ist;
Ausgeben (S1303) der biometrischen Informationen; und
Ausgeben (S1307), nachdem die Kommunikationsstörung aufgetreten ist, mindestens einer biometrischen Information, während die Signalverlustbenachrichtigung verzögert wird.

## Revendications

1. Procédé de fourniture d'une notification de perte de signal dans un système de surveillance de glucose, le procédé comprenant :
lorsqu'une défaillance de communication se produit entre un terminal et un émetteur de capteur, le retardement (S1003) d'une notification de perte de signal pendant une première période de temps par le terminal ;
la détermination (S1105) pour savoir si l'échec de communication persiste pendant la première période de temps par le terminal ;
lorsque l'échec de communication persiste pendant la première période de temps, le retardement (S1107) de la notification de perte de signal pendant une seconde période de temps suivie de la première période de temps ;
la détermination supplémentaire (S1109) pour savoir si la perte de signal persiste pendant la seconde période de temps par le terminal ;
lorsque la perte de signal persiste pendant la seconde période de temps, la génération (S1009) de la notification de perte de signal par le terminal ; et
l'émission (S1011), par le terminal, de la notification de perte de signal à un utilisateur.

2. Procédé selon la revendication 1, ladite génération (S1009) de la notification de perte de signal comprenant la génération de la notification de perte de signal en fonction d'un résultat de détermination pour savoir si la perte de signal persiste pendant la seconde période de temps.

3. Procédé selon la revendication 2, ladite détermination (S1109) pour savoir si la perte de signal persiste pendant la seconde période de temps comprenant la détermination de la persistance de la perte de signal en fonction de la réception de données du terminal à partir de l'émetteur de capteur pendant la seconde période de temps, et
ladite génération (S1009) de la notification de perte de signal comprenant la génération de la notification de perte de signal si la réception de données du terminal à partir de l'émetteur de capteur est arrêtée pendant la seconde période de temps.

4. Procédé selon la revendication 1, ladite détermination (S1105) de la persistance de l'échec de communication pendant la première période de temps comprenant la détermination de la persistance de l'échec de communication en fonction de l'échec de communication comprenant un cas dans lequel une connexion de communication entre le terminal et l'émetteur de capteur est perdue ou un cas dans lequel la réception de données du terminal depuis l'émetteur de capteur est arrêtée pendant que la connexion de communication est établie.

5. Procédé selon la revendication 1, ladite première période de temps étant plus courte que la seconde période de temps.

6. Procédé selon la revendication 1, ledit retardement (S1003) de la notification de perte de signal pendant la première période de temps comprenant l'émission d'informations biométriques pendant la première période de temps pendant laquelle la notification de perte de signal est retardée.

7. Procédé selon la revendication 1, comprenant en outre :
la réception (S1201), avant que la défaillance de communication ne se produise, d'une publicité provenant de l'émetteur de capteur ;
l'établissement (S1203) d'une connexion de communication avec l'émetteur de capteur ;
la demande (S1205) des informations biométriques provenant de l'émetteur de capteur en réponse à la publicité ; et
la réception (S1207) des informations biométriques provenant de l'émetteur de capteur.

8. Procédé selon la revendication 1, ladite publicité étant transmise de l'émetteur de capteur au terminal à chaque synchronisation de publicité pendant la première période de temps et la seconde période de temps, et
ladite première période de temps comprenant un plus petit nombre de synchronisations de publicité que les synchronisations de publicité comprises dans la seconde période de temps.

9. Procédé selon la revendication 1, comprenant en outre :
la réception (S1301), avant que la défaillance de communication ne se produise, d'informations biométriques provenant d'un émetteur de capteur fixé à un corps ;
l'émission (S1303) des informations biométriques ; et
l'émission (S1307), après que la défaillance de communication s'est produite, d'au moins une information biométrique tout en retardant la notification de perte de signal.
